# EUROPEAN PATENT APPLICATION

(11) **EP 1 004 309 A1**
(43) Date of publication of application: **31.05.2000**
(21) Application number: 98921787.2
(22) Date of filing: 22.05.1998
(51) Int. Cl.: A61K 31/55, C07D 495/14

(54) **ISRAPAFANT-CONTAINING WATER-BASE PREPARATIONS**

(30) Priority: 27.05.1997 JP 13706197; 27.05.1997 JP 15447497
(71) Applicant: Senju Pharmaceutical Co., Ltd., Osaka-shi, Osaka 541-0046 (JP); YOSHITOMI PHARMACEUTICAL INDUSTRIES, LTD., Osaka-shi Osaka 541-0046 (JP)
(72) Inventor: TERAI, Tadashi, Kobe-shi, Hyogo 658-0081 (JP); YASUEDA, Shinichi, Takarazuka-shi, Hyogo 665-0861 (JP); OGAWA, Takahiro, Nishinomiya-shi, Hyogo 662-0865 (JP); OTORI, Akira, Kobe-shi, Hyogo 673-0003 (JP)
(74) Representative: von Kreisler, Alek, Dipl.-Chem.
(86) International application number: JP9802275
(87) International publication number: WO9853828

(57) **Abstract**

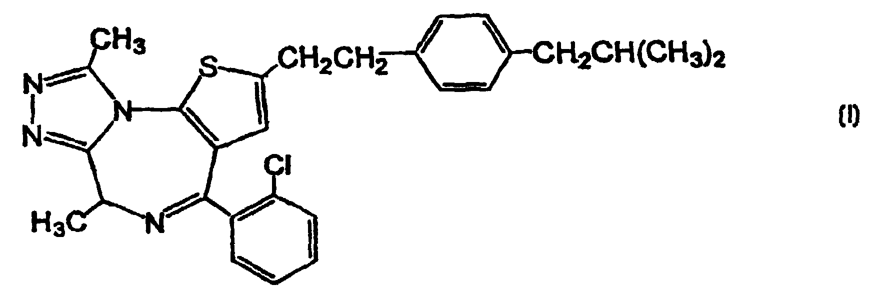

Water-base preparations comprising an aqueous solution or suspension of israpafant represented by structural formula (I) and providing eye drops and nose drugs exhibiting excellent therapeutic effects against allergic conjunctivitis and rhinitis by local administration.

## Description

### Technical Field

The present invention relates to an aqueous agent containing israpafant. More particularly, the present invention relates to an aqueous solution obtained by dissolving israpafant in water at a high concentration and a suspension obtained by dispersing israpafant stably in water. These aqueous agents are utilized as an eye drop or nasal drop particularly effective for allergic conjunctivitis, allergic rhinitis and the like.

### Background Art

4-(2-Chlorophenyl)-2-[2-(4-isobutylphenyl)ethyl]-6,9-dimethyl-6H-thieno[3,2-f][1,2,4]triazolo[4,3-a][1,4]diazepine (general name israpafant) disclosed in JP-B-5-55510 and having the formula (I) 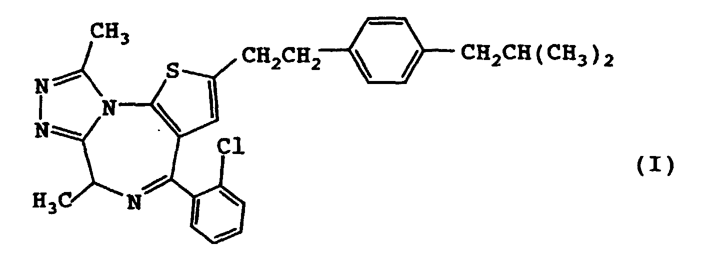 is known to show superior antagonistic action against platelet activating factor (PAF). Therefore, it is considered to be extremely useful for many diseases induced by PAF, such as inflammatory diseases, allergic diseases, anaphylactic shock, septic shock, vascular diseases such as DIC and the like, cardiac diseases, asthma, pulmonary edema, adult respiratory diseases, ulcer and the like.

In addition, DE 42 01 147 A1 discloses that a compound having a PAF antagonistic action and having the formula 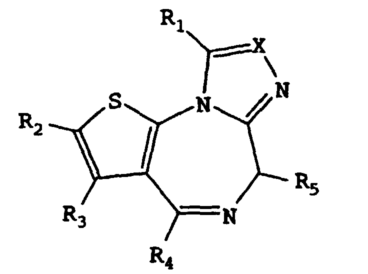 is useful for allergic conjunctivitis and further for allergic rhinitis, and recites some formulation examples of oral suspension containing this compound.

However, there is no disclosure of an aqueous preparation obtained by dissolving israpafant in water.

That is, israpafant is crystal or crystalline powder hardly soluble in water and easily dissolved in organic solvent such as dimethylformamide, methanol and the like. Nevertheless, the use of an organic solvent as a carrier of a preparation is inappropriate and, since in particular, an eye drop, a nasal drop and the like are locally administered to a site sensitive to irritation, the use of an organic solvent as a carrier to give an israpafant preparation should be avoided. As mentioned earlier, israpafant is hardly dissolved in water. Specifically, its solubility in water at 25°C is less than 0.01 w/v%, and the solubility in buffer (pH 4, 5, 6, 7, 8 and 9) is below detection limit (50 ng/ml) by HPLC, at such low concentration the efficacy of israpafant cannot be exerted in any way.

While to be mentioned later, when an israpafant suspension was orally administered to a guinea pig suffering from allergic conjunctivitis, no effect was observed in the test by the present inventors.

Therefore, there is a demand for an israpafant preparation which is effective for allergic conjunctivitis and further for allergic rhinitis.

### Disclosure of the Invention

It is therefore an object of the present invention to provide an aqueous agent containing israpafant, which is particularly effective against allergic conjunctivitis, allergic rhinitis and the like.

In particularl, an object of the present invention is to provide an aqueous preparation obtained by dissolving israpafant at a high concentration, namely, at a concentration at which its efficacy can be fully exerted, and a suspension preparation which is less irritative and which is capable of fully exerting the efficacy of israpafant.

Another object of the present invention is to provide an aqueous solution of israpafant, which is obtained by dissolving israpafant at a high concentration, and a method for producing a suspension.

The present inventors have conducted intensive studies in an attempt to achieve the above-mentioned problems, and found that israpafant can be dissolved in water at a high concentration in the presence of a surfactant and that this aqueous solution can be used as an eye drop and a nasal drop, which resulted in the completion of the present invention.

The present inventors have further found that administration of israpafant as an aqueous suspension to a local site of the eye or nose is effective for allergic conjunctivitis, allergic rhinitis, vernal conjunctivitis, conjunctival allergy caused by contact lens (giant papillary conjunctivitis), phlyctenular conjunctivitis, contact blepharocon junctivitis, Sjögren's syndrome, multiple corneal infiltration, keratitis disciformis, stromal keratitis, endothelium keratitis, episcleritis, scleritis, uveitis, retinal vasculitis, papillary vasulitis, optic neuritis, eosinophilic granuloma, rejection associated with keratoplastry, eye itching, sneeze, nose itching, hypersensitivity of the nose, nose vestibule eczema, anterior rhinitis sicca, nasal obstructioin and the like, and further that, since israpafant is a compound hardly soluble in water, it can be prepared into a stable aqueous eye drop or nasal drop suspension, which is free of agglomeration or caking of suspended particles, which resulted in the completion of the present invention.

Accordingly, the present invention provides the following.
(1) An aqueous agent containing israpafant, which is expressed by the formula (I): 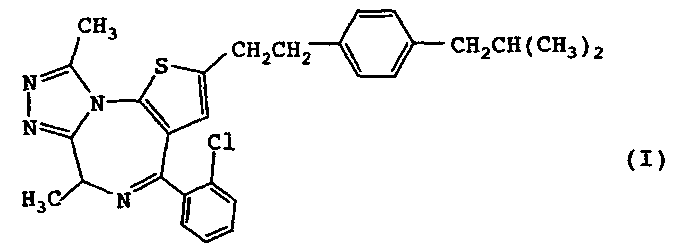
(2) The aqueous agent of (1) above, wherein the israpafant (I) has a concentration of not less than 0.01 w/v%.
(3) The aqueous agent of (2) above, wherein the aqueous agent is an eye drop or nasal drop.
(4) The aqueous agent of (3) above, wherein the eye drop or nasal drop is an aqueous solution containing israpafant (I).
(5) The aqueous agent of (4) above, wherein the israpafant (I) has a concentration of 0.01 - 0.1 w/v%.
(6) The aqueous agent of (5) above, further comprising a surfactant.
(7) The aqueous agent of (6) above, wherein the surfactant has a concentration of 0.5 - 10 w/v%.
(8) The aqueous agent of (7) above, wherein the surfactant is at least one member selected from the group consisting of a cationic surfactant, an anionic surfactant and a nonionic surfactant.
(9) The aqueous agent of (3) above, wherein the eye drop or nasal drop is a suspension containing israpafant (I).
(10) The aqueous agent of (9) above, wherein the israpafant has a concentration of 0.01 - 2 w/v%.
(11) The aqueous agent of (10) above, further comprising a suspending agent.
(12) The aqueous agent of (11) above, wherein the suspending agent is at least one member selected from a surfactant and a water soluble polymer.
(13) The aqueous agent of (12) above, wherein the surfactant has a concentration of 0.0001 - 0.1 w/v%.
(14) The aqueous agent of (13) above, wherein the surfactant is at least one surfactant selected from a nonionic surfactant and an anionic surfactant.
(15) The aqueous agent of (12) above, wherein the water soluble polymer has a concentration of 0.00001 - 0.5 w/v%.
(16) The aqueous agent of (15) above, wherein the water soluble polymer is a water soluble cellulose derivative.
(17) The aqueous agent of (16) above, wherein the water soluble cellulose derivative is at least one cellulose derivative selected from hydroxypropylmethylcellulose, carboxymethylcellulose and methylcellulose.
(18) The aqueous agent of (17) above, wherein the agent contains israpafant (I) in a proportion of 0.1 - 1 w/v% and hydroxypropyl methylcellulose in the weight ratio to israpafant (I) of 0.001 - 0.01:1.
(19) A method for producing an aqueous solution of israpafant, comprising dissolving israpafant of the formula (I): 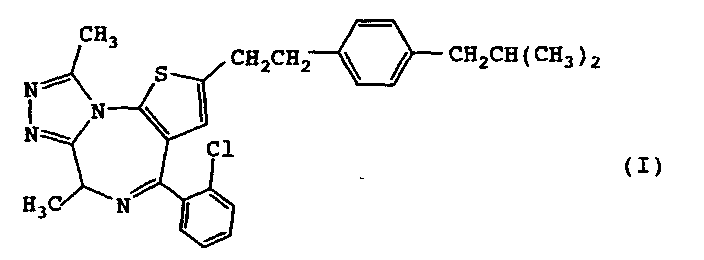 in water in the presence of a surfactant.
(20) A method for producing a suspension of israpafant, comprising suspending israpafant of the formula (I): 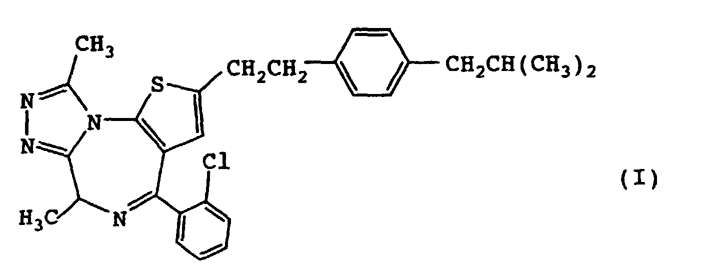 in water in the presence of a suspending agent.

Israpafant which is used as the active ingredient of the aqueous agent of the present invention is a compound expressed by the formula (I) 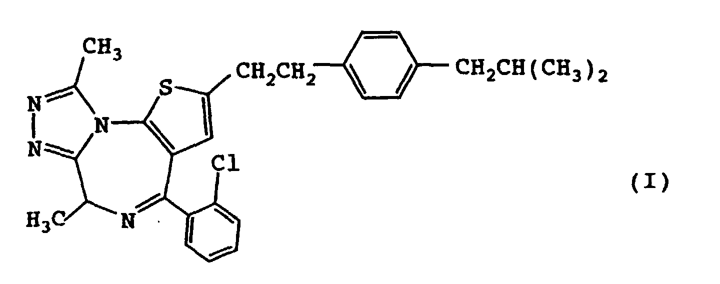 The concentration of israpafant (I) in the aqueous agent is appropriately determined, with preference given to not less than 0.01 w/v%. The aqueous agent of the present invention is preferably used as an eye drop or a nasal drop.

The aqueous israpafant solution of the present invention can be produced by dissolving israpafant in water in the presence of a surfactant.

Examples of the surfactant include cationic surfactants, anionic surfactants, nonionic surfactants and the like.

Examples of the cationic surfactant include benzalkonium salt such as benzalkonium chloride (BAK) and the like.

Examples of the anionic surfactant include sodium alkylsulfonate such as sodium dodecylsulfate (SDS), sodium pentanesulfonate, sodium octanesulfonate and the like.

The nonionic surfactant preferably has an HLB of 10 - 18 and a molecular weight of 500 - 4000, with preference given to ether nonionic surfactants and ester nonionic surfactants. Examples thereof include polyoxyethylene sorbitane fatty acid esters such as polysorbate 80 and the like, polyoxyethylene hydrogenated castor oil such as polyoxyethylene hydrogenated castor oil 60 (HCO 60) and the like, polyoxyethylene alkylphenyl formaldehyde condensate such as tyloxapol and the like, polyoxyethylene polyoxypropylene block copolymer such as poloxamer and the like, sucrose fatty acid ester and the like.

The above-mentioned surfactant can be used alone or in combination. In particular, since anionic surfactants cause irritation to the eye, when an aqueous solution containing an anionic surfactant is used as an eye drop, a nonionic surfactant is preferably used concurrently to reduce irritation.

The concentration of israpafant in the aqueous solution is not less than 0.01 w/v%, preferably 0.01 - 0.1 w/v%. When the concentration of israpafant is within the above-mentioned range, the local administration of an eye drop, a nasal drop and the like leads to exhibition of useful efficacy and preparation of an aqueous solution causing less irritation.

When dissolving israpafant in water in the above-mentioned range, the surfactant is preferably contained in 0.5 - 10 w/v% of the aqueous solution, more preferably 0.5 - 4 w/v%.

The proportion of the surfactant relative to israpafant is preferably 30 - 80 parts by weight, more preferably 45 - 60 parts by weight, of a cationic surfactant, 3 - 10 parts by weight, more preferably 4 - 7 parts by weight, of an anionic surfactant, and 50 - 200 parts by weight, more preferably 70 - 160 parts by weight, of a nonionic surfactant, per part by weight of israpafant.

Examples of the suspending agent to be used when preparing israpafant into an aqueous suspension in the present invention include a nonionic surfactant, an anionic surfactant, a water soluble polymer and the like, which can be used alone or in combination.

The nonionic surfactant preferably has an HLB of 10 - 18 and a molecular weight of 500 - 4000, with preference given to an ether type nonionic surfactant and an ester type nonionic surfactant.

Specific examples include polyoxyethylene sorbitane fatty acid esters such as polysorbate 80 and the like, polyoxyethylene hydrogenated castor oil such as polyoxyethylene hydrogenated castor oil 60 (HCO 60) and the like, polyoxyethylene alkylphenyl formaldehyde condensate such as tyloxapol and the like, polyoxyethylene polyoxypropylene block copolymer such as poloxamer and the like, sucrose fatty acid ester and the like. In addition, an anionic surfactant such as sodium lauryl sulfate (SDS) and the like can be used.

Preferable examples of the water soluble polymer include water soluble cellulose derivatives such as hydroxypropylmethylcellulose (HPMC), carboxymethylcellulose (CMC), methylcellulose (MC) and the like, carboxyvinyl polymer, macrogol, sodium chondroitin sulfate, polyvinylpyrrolidone such as polyvinylpyrrolidone K25 (PVP K25), polyvinylpyrrolidone K30 (PVP K30), polyvinylpyrrolidone K90 (PVP K90) and the like, polyvinyl alcohol and the like.

The concentration of israpafant in a suspension is generally 0.01 - 2 w/v%, preferably 0.1 - 1 w/v%. When the concentration of israpafant is within the above-mentioned range, useful efficacy for local administration can be exerted and a stable suspension can be prepared.

The suspending agent is contained in a proportion of 0.00001 - 0.5 w/v% in a suspension. The surfactant and water soluble polymer to be used as suspending agents can be used alone or in combination.

The concentration of each ingredient of the suspending agent is preferably 0.0001 - 0.1 w/v%, more preferably 0.001 - 0.1 w/v% and most preferably 0.005 - 0.1 w/v% in the case of a surfactant, and preferably 0.00001 - 0.5 w/v%, more preferably 0.0001 - 0.1 w/v% in the case of a water soluble polymer.

The proportion of israpafant and the surfactant is preferably 0.01 - 1 part by weight of a surfactant per part by weight of israpafant. The proportion of israpafant and the water soluble polymer is preferably 0.0001 - 1 part by weight, more preferably 0.0005 - 0.1 part by weight, and most preferably 0.001 - 0.01 part by weight, of a water soluble polymer per part by weight of israpafant. Particularly, when HPMC is used, a weight ratio of 0.001 - 0.01 parts by weight per part by weight of israpafant is preferable. When the concentration of the suspending agent is within the above-mentioned range, a stable suspension having fine dispersibility can be prepared.

The aqueous agent of the present invention can contain, where necessary, other anti-allergic agents, such as histamine release inhibitor, histamine receptor antagonist, leukotriene release inhibitor, leukotriene receptor antagonist, PAF release inhibitor, PAF receptor antagonist, IgE antibody production inhibitor, cytokine release inhibitor, cytokine receptor inhibitor and the like.

When the aqueous agent of the present invention is used as an eye drop or nasal drop, known additives typically used for an eye drop or a nasal drop can be used. Examples of such additive include isotonizing agent, buffer, chelating agent, preservative and the like.

Examples of isotonicity agent include inorganic salt such as sodium chloride, boric acid, potassium chloride and the like, and polyhydric alcohol such as glycerol, mannitol, sorbitol and the like.

The buffer may be, for example, borate buffer, phosphate buffer, acetate buffer, citrate buffer, Tris buffer, amino acid such as glutamine and ε-aminocapronic acid, and the like.

Examples of chelating agent include disodium edetate, citric acid and the like. Examples of preservative include quaternary ammonium salt such as benzalkonium chloride, benzetonium chloride and the like, p-aminobenzoate such as methyl p-aminobenzoate, ethyl p-aminobenzoate, propyl p-aminobenzoate, butyl p-aminobenzoate and the like, sorbic acid, chlorobutanol, disodium edetate, boric acid and the like.

The amounts of these additives are subject to no particular limitation, but an isotonicity agent is generally added in 0.5 - 6.5 w/v% of the aqueous agent, a buffer is generally added in 0.01 - 2 w/v% of the aqueous agent, a chelating agent is generally added in 0.001 - 1 w/v% of the aqueous agent, and a preservative is generally added in 0.001 - 2 w/v% of the aqueous agent.

The method for preparing an aqueous solution is subject to no particular limitation, and an aqueous solution can be obtained by various known methods. For example, a buffer, an isotonicity agent, a preservative and the like are added to sterile purified water for dissolution. Thereto are added a surfactant and then israpafant, and they are dissolved, where necessary, the solution may be heated to about 70°C. After cooling, a pH adjusting agent (hydrochloric acid, sodium hydroxide and the like) is added to adjust the pH to a desired value.

Alternatively, israpafant may be dissolved in a 0.5 - 10 w/v% cationic surfactant or anionic surfactant, and a buffer, an isotonizing agent, a preservative and the like are added and dissolved, whereafter the mixture is lyophilized, which lyophilization product is dissolved when in use in an injectable distilled water and the like, or israpafant, a buffer, an isotonicity agent, a preservative and the like are mixed in a mortar and the like, sealed in a vial and the like and dissolved when in use in a liquid for dissolution containing a surfactant.

The method for preparing a dispersion is not particularly limited and can be a method conventionally known. For example, a suspending agent, a buffer, an isotonicity agent and a preservative are added to sterile purified water and dissolved, during which the mixture may be heated. To this solution is added israpafant, which is uniformly suspended in various homogenizers, mixers, mills or by ultrasonication. Then, pH is adjusted using a pH adjusting agent (hydrochloric acid, sodium hydroxide and the like) to give an aqueous dispersion.

When the aqueous agent of the present invention thus prepared is used as an eye drop or nasal drop for the treatment of, for example, allergic conjunctivitis, allergic rhinitis, vernal conjunctivitis, conjunctival allergy caused by contact lens (giant papillary conjunctivitis), phlyctenular conjunctivitis, contact blepharocon junctivitis, Sjögren's syndrome, multiple corneal infiltration, keratitis disciformis, stromal keratitis, endothelium keratitis, episcleritis, scleritis, uveitis, retinal vasculitis, papillary vasulitis, optic neuritis, eosinophilic granuloma, rejection associated with keratoplastry, eye itching, sneeze, nose itching, hypersensitivity of the nose, nose vestibule eczema, anterior rhinitis sicca, nasal obstruction and the like, it is preferably administered 2 - 6 times a day by 20 - 50 µl per dose.

It is also possible to concurrently use the inventive agent with other anti-allergic, anti-inflammatory and/or anti-bacterial eye drop, nasal drop and the like.

### Brief Description of the Drawings

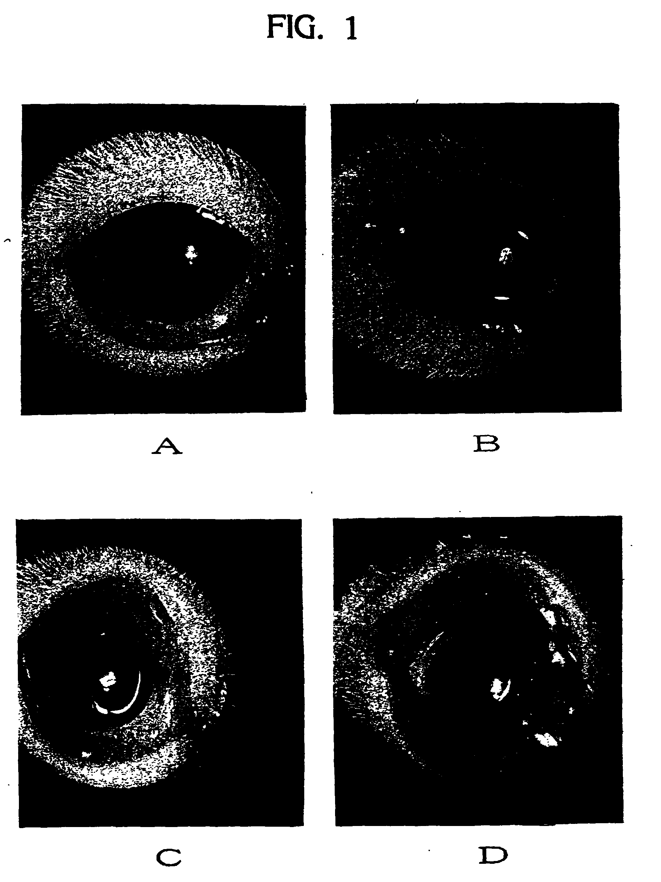

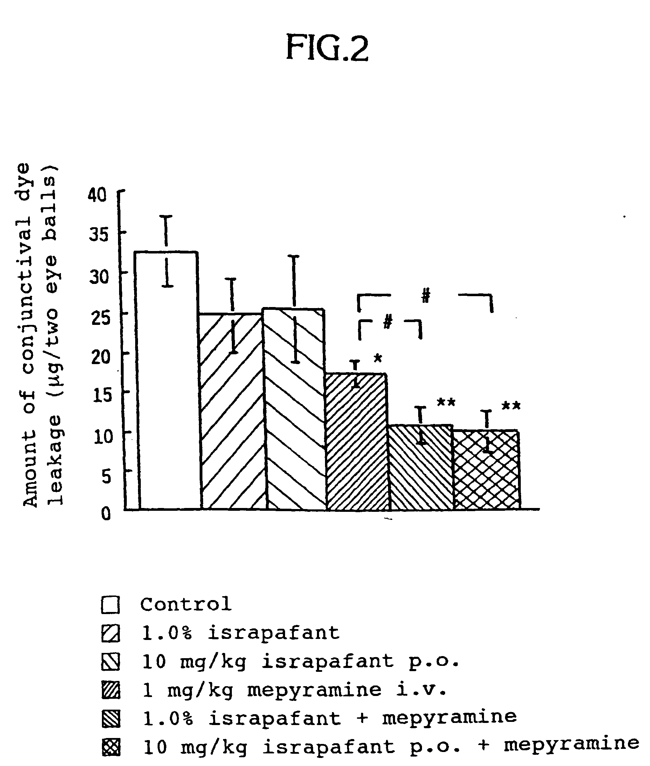

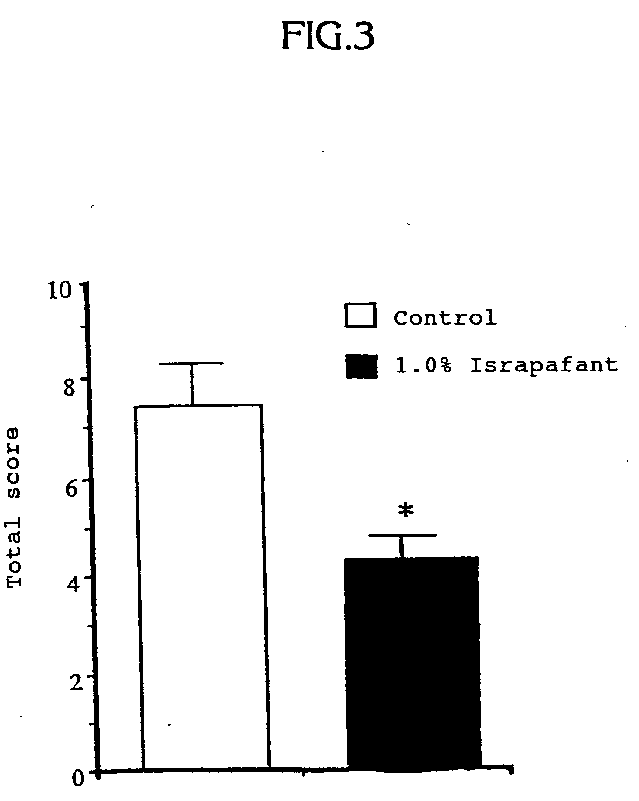

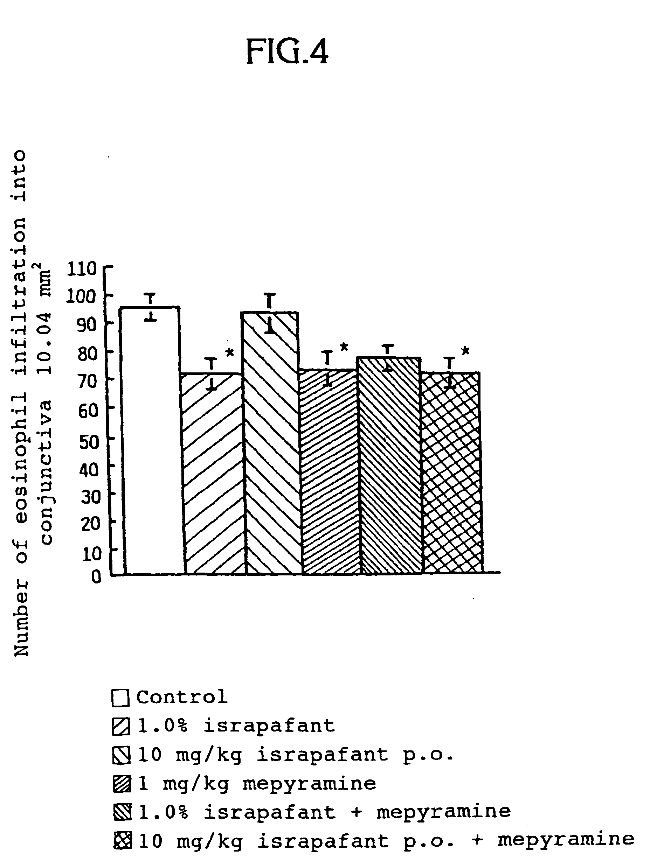

Fig. 1 is a photograph (biological morphology) showing the standard for grouping of guinea pigs for the evaluation of suppressive effect (early phase reaction) against promotion of conjunctival vascular permeability in Experimental Example 2; Fig. 2 shows conjunctival dye-leakage at 30 minutes after the second challenge for the evaluation of suppressive effect against promotion of conjunctival vascular permeability in Experimental Example 2; Fig. 3 shows scores of conjunctivitis at 20 minutes after the second challenge for the evaluation of suppressive effect against immediate allergic conjunctivitis in Experimental Example 2; and Fig. 4 shows number of conjunctival eosinophil infiltration at 6 hours after the second challenge for the evaluation of suppressive effect against conjunctival eosinophil infiltration in Experimental Example 2.

### Best Mode of Embodying the Invention

The present invention is explained in detail in the following by way of Examples and Experimental Examples, to which the present invention is not limited.

The dissolution test of israpafant is shown in the following.

### Experimental Example 1

Israpafant (manufactured by Yoshitomi Pharmaceutical Industries, Ltd.) was suspended in 0.1% acetate buffer (pH 4, 5, 6), phosphate buffer (pH 6, 7, 8) and borate buffer (pH 8, 9) in a concentration of 0.1% (w/v%, hereinafter the same). Each suspension was filled in a glass ampoule by 5 ml and shaken at 25°C overnight (ca. 15 hours). After passing through a 0.45 µm membrane filter, the solubility of israpafant was measured by HPLC.

In 0.1% phosphate buffer (pH 7) were dissolved the additives shown in Table 2 in a proportion of 0.5% (0.5% and 4% in the sole case of polysorbate). In the same manner as above, israpafant was suspended and dissolved, after which the solubility of israpafant was measured by HPLC.

### Quantitative Determination of israpafant - by HPLC

### HPLC conditions

column: YMC AM-302 (4.6×150 mm)
mobile phase: acetonitrile:0.1M ammonium acetate-87:13
detection: UV 244 nm
column temperature:35°C
flow rate: 0.7 ml/min
internal standard:1,3,5-triphenylbenzene

The solubility of the above-mentioned israpafant in each buffer is shown in Table 1, and the solubility in an aqueous solution containing various additives is shown in Table 2.

**Table 1**

| buffer | pH of buffer | solubility (µg/ml) |
|---|---|---|
| acetate buffer | pH 4 | 0.0 |
| acetate buffer | pH 5 | 0.0 |
| acetate buffer | pH 6 | 0.0 |
| phosphate buffer | pH 6 | 0.0 |
| phosphate buffer | pH 7 | 0.0 |
| phosphate buffer | pH 8 | 0.0 |
| borate buffer | pH 8 | 0.0 |
| borate buffer | pH 9 | 0.0 |

**Table 2**

| additive | solubility (µg/ml) |
|---|---|
| surfactant | |
| polysorbate 80 | 66.9 |
| polysorbate 80 (4% aq. solution) | 581.1 |
| HCO60 | 47.3 |
| BAK | 99.0 |
| SDS | 1057.0 |
| Tyloxapol | 29.5 |

| water soluble polymer | |
|---|---|
| HPMC | 0.6 |
| PVP(K30) | 0.0 |
| PVA(EG40) | 0.4 |
| CMC Na | 0.0 |
| chondroitin sulfate | 0.0 |
| PEG-1000 | 0.0 |
| PEG-4000 | 0.0 |

| cyclodextrin | |
|---|---|
| α-CD | 0.0 |
| β-CD | 0.0 |
| γ-CD | 0.0 |
| 2HP-β-CD | 0.0 |

| Others | |
|---|---|
| caffeine | 0.0 |
| propylene glycol | 0.0 |
| 2-pyrrolidone | 0.0 |

Each symbol in Table 2 is as follows.
HCO60:polyoxyethylene hydrogenated castor oil 60
BAK:benzalkonium chloride
SDS:sodium dodecylsulfate
HPMC:hydroxypropylmethylcellulose
PVP:polyvinylpyrrolidone
PVA:polyvinyl alcohol
CMC Na:sodium carboxymethylcellulose
PEG:polyethylene glycol
CD:cyclodextrin

The solubility of israpafant in each buffer was below detection limit (50 ng/ml) by HPLC. Israpafant did not dissolve in an aqueous solution containing a water soluble polymer or cyclodextrin. However, it dissolved well in an aqueous surfactant solution. Particularly, israpafant dissolved extremely well in a 4% aqueous polysorbate solution and a 0.5% aqueous sodium dodecylsulfate solution.

The effect of the eye drop of the present invention against allergic conjunctivitis, which is an ocular inflammation, is shown in the following.

### Experimental Example 2

### 1. Object

The suppressive effect of israpafant against guinea pig active sensitization allergic conjunctivitis was studied.

In allergic conjunctivitis, an early phase reaction that occurs 10 - 20 minutes after antigen challenge and late phase reaction that occurs 4-10 hours later have been clinically confirmed. In the former, histamine, leukotriene, PAF and the like liberated from mast cells are involved and conjunctivitis signs of itching, chemosis, injection and tearing are observed. In the latter, chemical mediators such as histamine, leukotriene, PAF and the like, and cytokine such as interleukin 3, interleukin 5 and the like are also involved, wherein edema and foreign body sensation are observed. It is said that eosinophils and lymphocytes infiltrate into conjunctiva and cause epithelial disorders at cornea and conjunctiva due to injurious proteins in eosinophil granules.

For investigation of usefulness of israpafant against allergic conjunctivitis, its suppressive effect against allergic conjunctivitis by guinea pig active sensitization was studied.

In this model with regard to the early phase reaction, promotion of conjunctival vascular permeability and signs of conjunctivitis were the index of evaluation, and for the late phase reaction, eosinophil infiltration into conjunctiva was the index of evaluation.

### 2. Animals used

Male Hartley guinea pigs weighing about 300 g purchased from Japan SLC. Ltd. were used. These animals had free access to a solid feed (γ ray irradiated feed LRC4, ORIENTAL YEAST CO., LTD.) and tap water, and were bred in a breeding chamber set to a temperature 23 ±2°C and humidity 55±15%.

### 3. Test drug

Israpafant (manufactured by Yoshitomi Pharmaceutical Industries, Ltd.) was suspended in a vehicle (pH 5) shown below at a concentration of 1% to give an eye drop suspension. In addition, a suspension of israpafant in 0.5% CMC at 10 mg/ml was prepared for oral administration. As a control, physiological saline was used.

| | |
|---|---|
| Polysorbate 80 | 0.1 g |
| sodium dihydrogenphosphate·2H₂O | 0.1 g |
| sodium chloride | 0.9 g |
| distilled water | 100 ml in total |

### 4. Test method

### 1) Suppressive effect (early phase reaction) against promotion of conjunctival vascular permeability

Guinea pigs were sensitized with a dispersion of 10 µg ovalbumin (OA) and 30 mg aluminum hydroxide gel by peritoneal administration. At 14 days after the sensitization, a 2.5% OA antigen solution was instilled into the both eyes by 10 µl to induce allergic conjunctivitis (first challenge). At 20 minutes after the last challenge, conjunctivitis signs were observed and the animals were grouped in such a manner that the severity of the condition was equalized from the degrees of chemosis, conjunctival injection and tearing.

The evaluation of a drug included the following steps. At 24 hours from the first challenge, 10 mg/kg Evans' blue was intravenously administered, and a 2.5% OA antigen solution was instilled into the both eyes by 10 µl to again induce conjunctivitis (the second challenge). Thirty minutes later, conjunctival dye-leakage part was excised, the parts of the both eyes were combined and the amount of the leaked dye was measured. The drug was instilled into the both eyes by 10 µl at 3, 2, 1 hour before the second challenge in the case of administration by instillation. In the case of an oral administration, 10 mg/kg was administered once at 1 hour before the second challenge. For suppression of histamine reaction, 1 mg/kg of mepyramine was intravenously administered one minute before.

### 2) Suppressive effect (early phase reaction) against immediate allergic conjunctivitis signs

In the same manner as in 1), sensitization and induction by the first challenge and the second challenge were performed. Twenty minutes later, conjunctivitis signs were observed, and chemosis was scored 0 - 6 points [see Fig. 1, A:0, B:2 (weak), C:4 (moderate), D:6 (strong)], conjunctival injection was scored 0 - 4 points (0: no injection, 1: very mild injection in the eye lid or bulbar conjunctiva, 2: mild injection in the eye lid or bulbar conjunctiva, 3: strong injection in the eye lid or bulbar conjunctiva, 4: strong injection in a wide area of the eye lid and bulbar conjunctiva), and tearing was scored 0 - 4 points (0: no tearing, 1: extremely small amount of tearing, 2: tearing, 3: a small amount of secretion, 4: a large amount of secretion), wherein evaluation followed the total scores. The drug was administered by instillation (10 µl) into one eye at 2, 4, 8 hours after the first challenge and 3, 2, 1 hour before the second challenge.

### 3) Suppressive effect (early phase reaction) against eosinophil infiltration into conjunctiva

In the same manner as in 1), sensitization and induction by first challenge and second challenge were performed.

The drug was evaluated by removing the eye ball at 6 hours after the second challenge, preparing an optical microscopic specimen according to a conventional method, staining eosinophils by Luna staining, and counting and averaging the number of eosinophils infiltrated into conjunctiva with regard to each of 6 visible areas (one visible area being 0.04 mm²) of the vicinity of conjunctival lymphoid tissue. The drug was administered in the same manner as in 1).

### test group

physiological saline (control group)
1.0% israpafant (instillation group)
10 mg/kg israpafant p.o. (oral group)
1 mg/kg mepyramine i.v. (intravenous injection group)
1.0% israpafant + 1 mg/kg mepyramine i.v. (concurrent instillation group)
10 mg/kg israpafant p.o.+1 mg/kg mepyramine i.v. (concurrent instillation group)

### 5. Results and discussion

### 1) Suppressive effect (early phase reaction) against promotion of conjunctival vascular permeability

Conjunctival dye-leakage at 30 minutes after the second challenge is shown in Fig. 2. In Fig. 2, each column shows mean± standard error (n=7 - 8). Significant difference from control group at *;p<0.05, **;p<0.01 (Dunnett's test). Significant difference from mepyramine group at #;p<0.05 (Student's test).

The dye-leakage (µg/two eye balls) of the control group instilled with physiological saline was 34.5 µg but that of the israpafant instillation group and oral administration group was 24.7 µg and 25.3 µg, respectively. Thus, they did not show a significant suppressive effect. The dye-leakage of the mepyramine administration group was 17.5 µg, thus showing significant suppression of promotion of vascular permeability. In contrast, concurrent instillation group and oral concurrent group showed less (11.0 µg and 10.1 µg, respectively) dye-leakage than the single administration group, thus significantly suppressing promotion of vascular permeability.

From the above results, it was suggested that israpafant was useful against early phase reaction when used concurrently with an antihistamic agent, because PAF is involved in the early phase reaction of allergic conjunctivitis.

### 2) Suppressive effect (early phase reaction) against immediate allergic conjunctivitis signs

The total score of conjunctivitis signs at 20 minutes after the second challenge is shown in Fig. 3. In Fig. 3, each column means mean ±standard error (n=8). Significant difference from control group at *;p<0.05 (Wilcoxon test).

When compared to the total score of the physiological saline instilled control group, 1% israpafant instillation group showed significant decrease in the total score.

From the above results, it was suggested that israpafant alleviated the conjunctivitis signs of immediate type allergy.

### 3) Suppressive effect (late phase reaction) against eosinophil infiltration into conjunctiva

The conjunctival eosinophil infiltration count at 6 hours after the second challenge is shown in Fig. 4. In Fig. 4, each column means mean±standard error (n=16). Significant difference from control group at *; p<0.05 (Dunnett's test).

The eosinophil infiltration count (cell/0.04 mm²) of the control group was 95.3, but it was 71.8 in the israpafant instillation group, showing significant suppression of eosinophil infiltration. In contrast, the oral group showed no suppressive effect. In addition, the mepyramine group, the concurrent instillation group and concurrent oral administration group showed suppressive effect, but the effect of each group was almost the same, and no effect from the concurrent use of israpafant was found.

From the above results, it was suggested that israpafant was useful against late phase reaction in view of significant suppression of eosinophil infiltration by single instillation. However, no effect was found by oral administration.

### Experimental Example 3

### 1. Object

The effect was studied when used as an eye drop of israpafant against rat PAF conjunctivitis.

### 2. Animal used

Male Wistar rats weighing about 130 g purchased from Clea Japan, Inc. were used. They were bred at temperature 23±2°C and humidity 55±15%.

### 3. Test drug

Israpafant was suspended at 0.03, 0.1, 0.3 and 1.0%. Physiological saline was used for the control group.

The formulation of the vehicle for an israpafant eye drop was as shown below, wherein the pH of the eye drop at each concentration was 7.0.

| | |
|---|---|
| sodium dihydrogenphosphate·2H₂O | 0.1 g |
| sodium chloride | 0.9 g |
| polysorbate 80 | 0.1 g |
| distilled water | 100 ml in total |

### 4. Test method

PAF diluted to 4 µg/ml was administered to the upper subconjunctiva of the rat by 25 µl and at the same time, 0.5% Evans' blue (0.5 ml) was intravenously administered. Thirty minutes later, the dye-leakage part of the conjunctiva was excised and the dye was extracted with formamide, after which absorbance (625 nm) was measured. The test drug was administered by instillation (5 µl) to the eye at 1, 2 hours before subconjunctival induction of PAF.

### 5. Results and Discussion

The dye-leakage of each group is shown in Table 3.

**Table 3**

| drug | concentration (%) | dose (µg) | n | amount of dye leakage (µg/site) | inhibition (%) |
|---|---|---|---|---|---|
| physiological saline | - | - | 8 | 40.03±2.57 | - |
| israpafant | 0.03 | 3 | 8 | 32.29±3.31 | 19.1 |
| | 0.1 | 10 | 8 | 28.17±1.42* | 29.6 |
| | 0.3 | 30 | 7 | 23.37±2.56* | 41.6 |
| | 1.0 | 100 | 7 | 18.74±2.20* | 53.2 |
| Each value represents the mean±standard error. Significant difference from physiological saline at *;p<0.01 (Dunnett's test). | | | | | |

The dye-leakage of the control group was 40.0 µg/site but israpafant showed a concentration-dependent suppressive effect, wherein the suppressive percentage at 0.03% - 1.0% concentration was 19.1%, 29.6%, 41.6% and 53.2%, showing significant difference at a concentration of 0.1% or greater. From the above results, israpafant showed a concentration-dependent suppressive effect against promotion of vascular permeability at conjunctival local site by PAF, demonstrating good penetration into a tissue of the present preparation.

### Experimental Example 4

### 1. Object

The stability of israpafant suspension was studied.

### 2. Drug and reagent

Polysorbate 80 used met the standard of the Japan Pharmacopoeia.

### 3. Test method

The following israpafant suspension formulation was prepared. Polysorbate 80 was used as the suspending agent, phosphate buffer (pH 7) was used as the buffer, and sodium chloride was used as the isotonizing agent. The suspension was filled in glass ampoules by 5 ml and stored at 25, 40 and 60°C. The suspension was sampled with time (3 days later, one week later, 2 weeks later) and israpafant content was quantitatively determined by HPLC.

| | |
|---|---|
| israpafant | 0.1 g |
| polysorbate 80 | 0.1 g |
| sodium dihydrogenphosphate·2H₂O | 0.1 g |
| sodium chloride | 0.9 g |
| distilled water | 100 ml in total |

### Quantitative Determination of israpafant - by HPLC

### HPLC conditions

column: YMCAM-302 (4.6×150 mm)
mobile phase: acetonitrile:0.1M ammonium acetate=87:13
detection: UV 244 nm
column temperature:35°C
flow rate: 0.7 ml/min
internal standard:1,3,5-triphenyl benzene

### 4. Results and Discussion

The results are shown in Table 4.

**Table 4**

| temperature (°C) | residual content (%) | | | |
|---|---|---|---|---|
| | initial | 3 days later | 1 week later | 2 weeks later |
| 25 | 100.0 (pH 6.97) | - | - | 101.4 (pH 6.94) |
| 40 | | - | - | 99.7 (pH 6.94) |
| 60 | | 97.3 (pH 6.96) | 97.3 (pH 6.97) | 100.3 (pH 6.96) |
| 80 | | 99.3 (pH 6.97) | - | - |

As is evident from the Table, the 0.1% israpafant suspension after storage at 60°C for 2 weeks showed an israpafant residual content of 100.3%. The residual content after storage at 80°C for 3 days was 99.3%, demonstrating the stability of the 0.1% israpafant dispersion.

### Example 1 (Formulation Example of eye drop)

Using the following ingredients, sodium dodecylsulfate, polysorbate 80, sodium dihydrogenphosphate dihydrate, boric acid and benzalkonium chloride were dissolved in sterile purified water. Using a pH adjusting agent, the pH was adjusted to about 7. Israpafant was added and the mixture was heated to about 70°C to allow dissolution. After cooling, the pH adjusting agent was used again to adjust the pH 7 to give an eye drop.

| | |
|---|---|
| israpafant | 0.1 g |
| sodium dodecylsulfate | 0.65 g |
| polysorbate 80 | 3.25 g |
| sodium dihydrogenphosphate dihydrate | 0.1 g |
| boric acid | 1.6 g |
| benzalkonium chloride | 0.005 g |
| sodium hydroxide | q.s. |
| sterile purified water | amount to make the total amount 100 ml |
| pH | 7.0 |

### Example 2 (Formulation Example of nasal drop)

Using the following ingredients and in the same manner as in Example 1, a nasal drop was obtained.

| | |
|---|---|
| israpafant | 0.05 g |
| polysorbate 80 | 4.0 g |
| sodium dihydrogenphosphate dihydrate | 0.1 g |
| sodium chloride | 0.9 g |
| benzalkonium chloride | 0.01 g |
| sodium hydroxide | q.s. |
| sterile purified water | amount to make the total amount 100 ml |
| pH | 8.0 |

### Example 3 (Formulation Example of eye drop)

Using the following ingredients and in the same manner as in Example 1, an eye drop was obtained.

| | |
|---|---|
| israpafant | 0.05 g |
| sodium octanesulfonate | 0.5 g |
| polysorbate 80 | 3.35 g |
| sodium dihydrogenphosphate dihydrate | 0.1 g |
| sodium chloride | 0.9 g |
| benzalkonium chloride | 0.005 g |
| sodium hydroxide | q.s. |
| sterile purified water | amount to make the total amount 100 ml |
| pH | 7.0 |

### Example 4 (Formulation Example of eye drop)

Using the following ingredients and in the same manner as in Example 1, an eye drop was obtained.

| | |
|---|---|
| israpafant | 0.05 g |
| sodium pentanesulfonate | 0.5 g |
| polysorbate 80 | 4.15 g |
| sodium dihydrogenphosphate dihydrate | 0.1 g |
| conc. glycerol | 2.6 g |
| benzalkonium chloride | 0.005 g |
| sodium hydroxide | q.s. |
| sterile purified water | amount to make the total amount 100 ml |
| pH | 7.0 |

### Example 5 (Formulation Example of eye drop)

Using the following ingredients and in the same manner as in Example 1, an eye drop was obtained.

| | |
|---|---|
| israpafant | 0.1 g |
| SDS | 0.65 g |
| polysorbate 80 | 3.25 g |
| sodium acetate | 0.1 g |
| sodium chloride | 0.9 g |
| methyl p-hydroxybenzoate | 0.026 g |
| propyl p-hydroxybenzoate | 0.014 g |
| hydrochloric acid | q.s. |
| sterile purified water | amount to make the total amount 100 ml |
| pH | 5.0 |

### Example 6

| | |
|---|---|
| israpafant | 0.1 g |
| polysorbate 80 | 0.1 g |
| monosodium phosphate dihydrate | 0.1 g |
| sodium chloride | 0.9 g |
| benzalkonium chloride | 0.005 g |
| sodium hydroxide | appropriate amount |
| sterile purified water | amount to make the total amount 100 ml |
| pH | 7.0 |

### Example 7

| | |
|---|---|
| israpafant | 1.0 g |
| HPMC | 0.001 g |
| sodium dihydrogenphosphate dihydrate | 0.1 g |
| sodium chloride | 0.9 g |
| benzalkonium chloride | 0.005 g |
| sodium hydroxide | appropriate amount |
| sterile purified water | amount to make the total amount 100 ml |
| pH | 7.0 |

### Example 8

| | |
|---|---|
| israpafant | 0.1 g |
| HCO-60 | 0.1 g |
| sodium dihydrogenphosphate dihydrate | 0.1 g |
| sodium chloride | 0.9 g |
| benzalkonium chloride | 0.005 g |
| sodium hydroxide | appropriate amount |
| sterile purified water | amount to make the total amount 100 ml |
| pH | 7.0 |

### Example 9

| | |
|---|---|
| israpafant | 1.0 g |
| HPMC | 0.01 g |
| sodium dihydrogenphosphate dihydrate | 0.1 g |
| sodium chloride | 0.9 g |
| methyl p-hydroxybenzoate | 0.026 g |
| propyl p-hydroxybenzoate | 0.014 g |
| sodium hydroxide | appropriate amount |
| sterile purified water | amount to make the total amount 100 ml |
| pH | 5.0 |

### Example 10

| | |
|---|---|
| israpafant | 1.0 g |
| HPMC | 0.01 g |
| boric acid | 1.6 g |
| benzalkonium chloride | 0.005 g |
| sodium hydroxide | appropriate amount |
| sterile purified water | amount to make the total amount 100 ml |
| pH | 7.0 |

### Example 11

| | |
|---|---|
| israpafant | 0.1 g |
| tyloxapol | 0.1 g |
| sodium dihydrogenphosphate dihydrate | 0.1 g |
| sodium chloride | 0.9 g |
| benzalkonium chloride | 0.005 g |
| sodium hydroxide | appropriate amount |
| sterile purified water | amount to make the total amount 100 ml |
| pH | 7.0 |

### Example 12

| | |
|---|---|
| israpafant | 0.1 g |
| SDS | 0.001 g |
| sodium dihydrogenphosphate dihydrate | 0.1 g |
| boric acid | 1.6 g |
| benzalkonium chloride | 0.005 g |
| sodium hydroxide | appropriate amount |
| sterile purified water | amount to make the total amount 100 ml |
| pH | 7.0 |

### Example 13

| | |
|---|---|
| israpafant | 1.0 g |
| methylcellulose | 0.001 g |
| sodium dihydrogenphosphate dihydrate | 0.1 g |
| sodium chloride | 0.9 g |
| benzalkonium chloride | 0.005 g |
| sodium hydroxide | appropriate amount |
| sterile purified water | amount to make the total amount 100 ml |
| pH | 7.0 |

### Example 14

| | |
|---|---|
| israpafant | 0.1 g |
| methylcellulose | 0.1 g |
| sodium acetate | 0.1 g |
| conc. glycerol | 2.6 g |
| methyl p-hydroxybenzoate | 0.026 g |
| propyl p-hydroxybenzoate | 0.014 g |
| chlorobutanol | 0.2 g |
| sodium hydroxide | appropriate amount |
| sterile purified water | amount to make the total amount 100 ml |
| pH | 5.0 |

### Example 15

| | |
|---|---|
| israpafant | 0.1 g |
| sucrose fatty acid ester | 0.05 g |
| sodium dihydrogenphosphate dihydrate | 0.1 g |
| sodium chloride | 0.9 g |
| benzalkonium chloride | 0.005 g |
| sodium hydroxide | appropriate amount |
| sterile purified water | amount to make the total amount 100 ml |
| pH | 7.0 |

### Example 16

| | |
|---|---|
| israpafant | 0.1 g |
| polysorbate 80 | 0.005 g |
| PVP K30 | 0.2 g |
| sodium dihydrogenphosphate dihydrate | 0.1 g |
| sodium chloride | 0.9 g |
| benzalkonium chloride | 0.005 g |
| sodium hydroxide | appropriate amount |
| sterile purified water | amount to make the total amount 100 ml |
| pH | 7.0 |

### Example 17

| | |
|---|---|
| israpafant | 1.0 g |
| HPMC(2910) | 0.004 g |
| sodium dihydrogenphosphate dihydrate | 0.1 g |
| sodium chloride | 0.9 g |
| benzalkonium chloride | 0.005 g |
| sodium hydroxide | appropriate amount |
| sterile purified water | amount to make the total amount 100 ml |
| pH | 7.0 |

### Industrial Applicability

The aqueous agent of the present invention shows superior therapeutic effect when locally administered as an eye drop or a nasal drop for allergic conjunctivitis, allergic rhinitis, venal conjunctivitis, conjunctival allergy caused by contact lens (giant papillary conjunctivitis), phlyctenular conjunctivitis, contact blepharoconjunctivitis, Sjögren's syndrome, multiple corneal infiltration, keratitis disciformis, stromal keratitis, endothelium keratitis, episcleritis, scleritis, uveitis, retinal vasculitis, papillary vasulitis, optic neuritis, eosinophilic granuloma, rejection associated with keratoplastry, eye itching, sneeze, nose itching, hypersensitivity of the nose, nose vestibule eczema, anterior rhinitis sicca, nasal obstruction and the like.

This application is based on patent application Nos. 137061/1997 and 154474/1997 filed in Japan, the contents of which are hereby incorporated by reference.

## Claims

1. An aqueous agent containing israpafant, which is expressed by the formula (I):

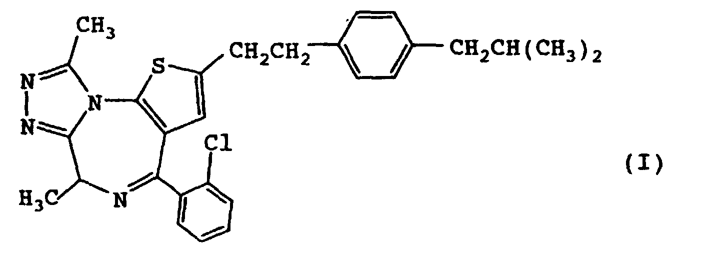

2. The aqueous agent of claim 1, wherein the israpafant (I) has a concentration of not less than 0.01 w/v%.

3. The aqueous agent of claim 2, wherein the aqueous agent is an eye drop or nasal drop.

4. The aqueous agent of claim 3, wherein the eye drop or nasal drop is an aqueous solution containing israpafant (I).

5. The aqueous agent of claim 4, wherein the israpafant (I) has a concentration of 0.01 - 0.1 w/v%.

6. The aqueous agent of claim 5, further comprising a surfactant.

7. The aqueous agent of claim 6, wherein the surfactant has a concentration of 0.5 - 10 w/v%.

8. The aqueous agent of claim 7, wherein the surfactant is at least one member selected from the group consisting of a cationic surfactant, an anionic surfactant and a nonionic surfactant.

9. The aqueous agent of claim 3, wherein the eye drop or nasal drop is a suspension containing israpafant (I).

10. The aqueous agent of claim 9, wherein the israpafant has a concentration of 0.01 - 2 w/v%.

11. The aqueous agent of claim 10, further comprising a suspending agent.

12. The aqueous agent of claim 11, wherein the suspending agent is at least one member selected from a surfactant and a water soluble polymer.

13. The aqueous agent of claim 12, wherein the surfactant has a concentration of 0.0001 - 0.1 w/v%.

14. The aqueous agent of claim 13, wherein the surfactant is at least one member selected from a nonionic surfactant and an anionic surfactant.

15. The aqueous agent of claim 12, wherein the water soluble polymer has a concentration of 0.00001 - 0.5 w/v%.

16. The aqueous agent of claim 15, wherein the water soluble polymer is a water soluble cellulose derivative.

17. The aqueous agent of claim 16, wherein the water soluble cellulose derivative is at least one cellulose derivative selected from hydroxypropylmethylcellulose, carboxymethylcellulose and methylcellulose.

18. The aqueous agent of claim 17, wherein the agent comprises israpafant (I) in a proportion of 0.1 - 1 w/v% and hydroxypropylmethylcellulose in the weight ratio to israpafant (I) of 0.001 - 0.01:1.

19. A method for producing an aqueous solution of israpafant, comprising dissolving israpafant of the formula (I): 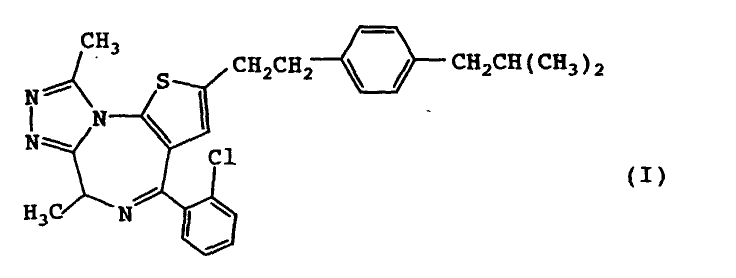 in water in the presence of a surfactant.

20. A method for producing a suspension of israpafant, comprising suspending israpafant of the formula (I): 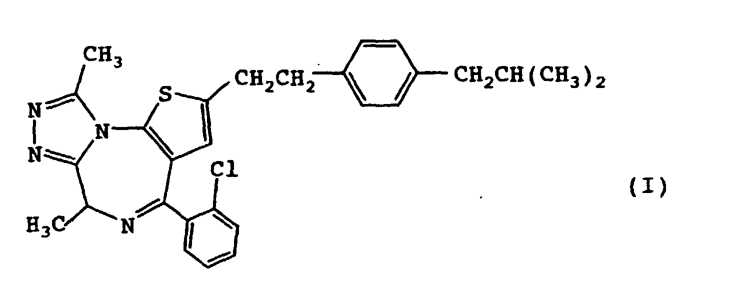 in water in the presence of a suspending agent.
